# EUROPEAN PATENT APPLICATION

(11) **EP 4 105 327 A1**
(43) Date of publication of application: **21.12.2022**
(21) Application number: 21887873.4
(22) Date of filing: 29.04.2021
(51) Int. Cl.: C12N 15/10, C12N 15/09, C12N 15/11, C12Q 1/6844, C12Q 1/6848, C12Q 1/68

(54) **METHOD FOR SYNTHESIZING NUCLEIC ACID UNDER CONSTANT TEMPERATURE CONDITIONS, KIT, AND APPLICATION**

(71) Applicant: Ningbo Institute of Life and Health Industry University of Chinese Academy of Sciences, Jiangbei District Ningbo, Zhejiang 315016 (CN)
(72) Inventor: MAO, Rui, Ningbo, Zhejiang 315016 (CN); WANG, Tianzuo, Ningbo, Zhejiang 315016 (CN); CAI, Ting, Ningbo, Zhejiang 315016 (CN)
(74) Representative: karo IP
(86) International application number: PCT/CN2021/090884
(87) International publication number: WO 2022/226870

(57) **Abstract**

Disclosed herein is a method for the synthesis of nucleic acids under constant-temperature conditions and a kit and use thereof. The method includes the steps of providing a nucleic acid consisting of M1c, F1c, M, R1, and M2c regions in sequence in the 3' to 5' direction, wherein the M region comprises M1 and M2 regions, and a closed-loop structure is formed through annealing of the M2c region at the 5' end and the M1c region at the 3' end of the nucleic acid to the M region on the same strand; and conducting reaction with the nucleic acid itself as a template for continuous elongation of the nucleic acid strand through annealing of primers, first oligonucleotide I and second oligonucleotide II, to the Flc region and the Rlc region of the nucleic acid, respectively. According to the method for synthesis of nucleic acids in the present invention, the core primer is about 30 bp in length, which is 10 bp shorter than the core primer of about 40 bp for LAMP and CAMP, thus saving about 25% in the cost of the primer and also increasing the reaction rate for the synthesis of nucleic acid.

## Description

### TECHNICAL FIELD

The present invention relates to the technical field of genetic engineering, in particular to a method for the synthesis of nucleic acids under constant-temperature conditions and a kit and use thereof.

### BACKGROUND

The genetic characteristics carried by genes can be directly analyzed by nucleotide sequence complementarity analysis method on the basis of the complementary base-pairing rules proposed by Watson and Crick in 1950s. This analysis is a very powerful method to identify genetic diseases, cancer, and microorganisms. However, it is generally difficult to detect a very small amount of target genes in a sample, and the target genes must be amplified or their detection signals are amplified. PCR (polymerase chain reaction) is considered to be the most classical method for amplifying a target gene (Saiki, Gelfand et al., 1988), and is also the most commonly used technique for in vitro nucleic acid sequence amplification. The major problems with this approach are encountered: a special program temperature control system must be used in actual operation; the sample and reaction solution are susceptible to external contamination, and false positive problems are prominent. For example, if a complementary strand is accidentally synthesized mistakenly in PCR, and the product serves as a template in the following reactions, resulting in erroneous results.

On the other hand, compared with the cumbersome temperature-controlled process for the synthesis of a nucleic acid, techniques for the synthesis of a nucleic acid under constant-temperature conditions are also developed by scientists (Zhao, Chen et al., 2015), such as nucleic acid sequence-based amplification (NASBA), rolling circle amplification (RCA), strand displacement amplification (SDA), loop-mediated isothermal amplification (LAMP), helicase-dependent amplification (HDA), recombinase polymerase amplification (RPA), and competitive annealing mediated isothermal amplification (CAMP), etc..

NASBA, also known as TMA (transcription-mediated amplification process), has no need for complex temperature control. NASBA requires a thermal denaturation step until that double-stranded DNA is formed; however, the subsequent transcription reaction proceeds with T7 RNA polymerase under constant-temperature conditions. The combination of multiple enzymes such as reverse transcriptase, RNase H, reverse transcriptase, and T7 RNA polymerase is necessary and relatively high in cost. At the same time, this process is high in cost due to the complex reaction conditions for multiple enzymes, and this process is hard to promote because of the tedious operation.

RCA (Rolling Circle Amplification) aims to mimic the rolling circle replication process of circular DNA in microorganisms, and in the case of circular single-stranded DNA templates, and the primers binding to the template can only achieve amplification of circular nucleic acids in the original method. This method also has the disadvantages of requirement of multiple enzymes and long amplification time.

SDA (Strand Displacement Amplification) is also known to amplify a template DNA having a sequence complementary to a target sequence (Zhang, Cui et al., 1992). The amplified product of SDA differs in structure from the native nucleic acid and there is a limit to the use of restriction enzymes for cleavage or applying the amplified product to gene cloning. This is also a major contributor to higher costs. Furthermore, there is a non-specific signaling problem with this approach due to the disintegration of restriction enzymes.

HDA (Helicase-dependent Isothermal DNA Amplification) is a new nucleic acid isothermal amplification technique developed by researchers of NEB Corporation, USA, in 2004 (Vincent, Xu et al. 2004). This technique mimics the natural process of DNA replication in natural organisms, wherein DNA duplexes are unwound by use of the helicase under constant temperature conditions, while single-stranded DNA-binding protein (SSB) is used to stabilize the unwound single-strand and provides binding templates for primers, thus synthesizing complementary strands by catalysis of DNA polymerase. The newly synthesized double strands are further unwound into single strands under the action of the helicase and enter the above-mentioned cyclic amplification reaction as a template for the next round of synthesis, finally achieving exponential growth of the target sequence.

RPA (Recombinase Polymerase Amplification) or RAA (Recombinase aid amplification) are two similar methods for isothermal amplification of nucleic acids based on recombinant polymerases. Recombinase µvsX from bacteriophage is used for RPA, while a recombinase from bacteria or fungi is used for RAA. The RAA and RPA are rapid in reactions and can provide detectable levels of amplified products within ten minutes. However, it is necessary to select primers having good specificity and being capable of binding to recombinases in the whole process, and at the same time, the use of three enzymes will greatly increases its cost and makes the design of primers difficult.

The core of LAMP technique (Notomi, Okayama et al., 2000) is that four specific primers are designed for six regions on the target gene and a highly active strand displacement DNA polymerase is employed, allowing continuous self-cycling of strand displacement DNA synthesis. One of the limitations of this technique is a tedious process because the high specificity and sensitivity depend on the properties of four primers, and the optimal primers usually need to be obtained through sequence alignment, online primer design, primer screening, and specificity test.

CAMP (Patent Application NO. 201710828028.8) is a process of re-designing and planning the working mode of primers on the basis of PCR primers, forming a competitive stem-loop initial amplification structure, designing the formation process of the structural loop, and further achieving the synthesis of nucleic acids (CN107446919B).

### SUMMARY

The present invention aims to provide a method for the synthesis of nucleic acids under constant-temperature conditions with a single enzyme. The method involves a new principle inspired by the common duplex helix structure of DNA, molecular beacon probes, LAMP and CAMP, a new working model of primers is re-designed on the basis of PCR primers, a closed stem-loop initial amplification structure is formed, the process of forming a structural loop is designed and optimized, and the outer primer may be optionally used. One advantage of the present invention is that the core primer used is about 30 bp in length, which is 10 bp shorter than the core primer of about 40 bp for LAMP and CAMP, thus saving about 25% of the cost of the primer. The present invention utilizes a polymerase to catalyze strand displacement-type complementary strand synthesis without complicated temperature control, which is beneficial for the synthesis of nucleic acid. DNA polymerase is an enzyme used in methods such as SDA, RCA, LAMP, CAMP, etc. In addition, the present invention can significantly improve the rate of the synthesis of nucleic acids compared to the CAMP technique.

The present invention improves the supply of 3'-OH in the known method, and it is found that the 3'-OH structure can be provided by using oligonucleotides having a specific structure without any additional enzymatic reaction, thereby realizing the present invention. That is, the present invention relates to a method for the synthesis of nucleic acids, a method for amplification of nucleic acids by using the synthesis method of nucleic acids, and a kit for the synthesis of nucleic acids using the method.

Specific technical solutions of the present invention are as follows:
A non-diagnostic method for the synthesis of nucleic acids under constant-temperature conditions comprises the steps of:
1) providing a nucleic acid consisting of M1c, F1c, M, R1, and M2c regions in sequence in the 3' to 5' direction, wherein the M region comprises M1 and M2 regions, and the M2c region at the 5' end and the M1c region at the 3' end of the nucleic acid are complementary to M2 region and M1 region respectively; and a closed stem-loop structure is formed through annealing of the M2c region at the 5' end and the M1c region at the 3' end of the nucleic acid to the M region on the same strand;
2) annealing a first oligonucleotide I to the Flc region of the nucleic acid provided in step 1), and then performing synthesis with the F1 region of the first oligonucleotide I as the starting point for synthesis; wherein the first oligonucleotide I comprises an M1 region and an F1 region;
3) annealing the M1c region at the 3' end of nucleic acid provided in step 1) to the M1 region, and synthesizing a complementary strand of the nucleic acid with the nucleic acid as a template, the synthesized nucleic acid sequence is referred to as nucleic acid A;
4) annealing a second oligonucleotide II to the Rlc region of the nucleic acid A provided in step 3), and then performing synthesis with the R1 region of the second oligonucleotide II as the starting point for synthesis; wherein the second oligonucleotide II comprises an R1 region and an M2c region; and
5) annealing the M region at the 3' end of the nucleic acid A provided in step 3) to the adjacent Mc region, and synthesizing a complementary strand of the nucleic acid A with the nucleic acid A as a template.

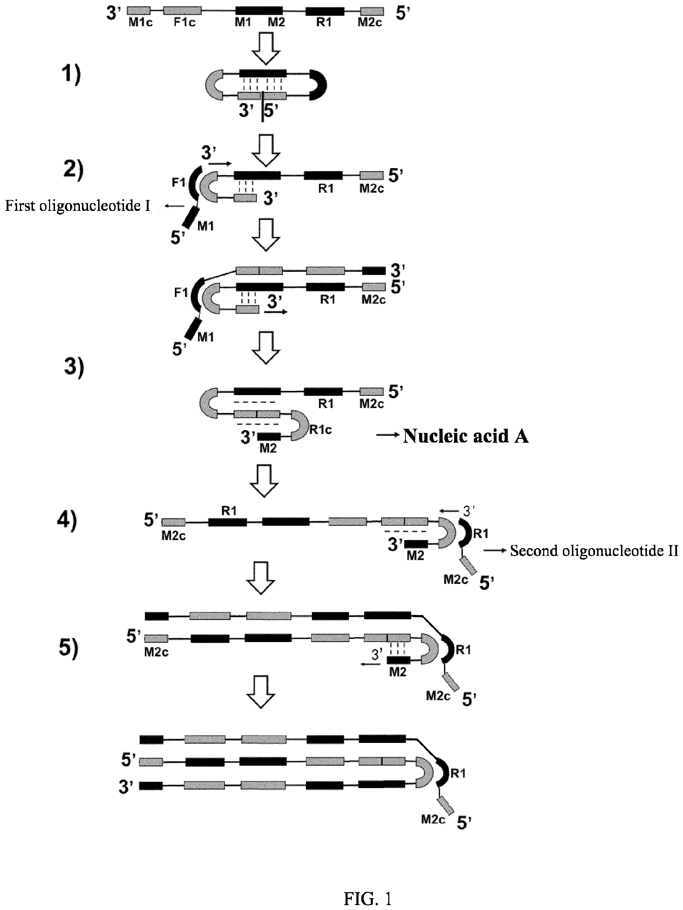

With reference to FIG. 1, which shows a schematic representation of the synthesis of nucleic acids of the present invention. The constant temperature in the present invention means that the whole reaction process involves synthesizing within a temperature range of 60-65 °C.

As a preferred embodiment, the polymerase used in the nucleic acid reactions of the present invention comprises one or more of Bst DNA polymerase, Bca (exo-)DNA polymerase, DNA polymerase I Klenow fragment, Vent DNA polymerase, Vent (Exo-) DNA polymerase (Vent DNA polymerase lacking exonuclease activity), Deep Vent DNA polymerase, Deep Vent (Exo-) DNA polymerase (Deep Vent DNA polymerase lacking exonuclease activity), Φ29 phage DNA polymerase, MS-2 phage DNA polymerase, and the like. Bst DNA polymerase or Bst 2.0 DNA polymerase is preferably used.

An unwinding temperature regulator, preferably betaine, may be added to the nucleic acid reaction of the present invention, more preferably, the betaine in the reaction solution has a concentration of 0.2-3.0 M.

As a preferred embodiment, the resulting nucleic acid strand is capable of indefinite elongation by self-pairing, and the M1c region at the 3' end of the nucleic acid strand will be paired with the complementary segment, the M1 region, on the strand to serve as the starting point for synthesis to continuously elongate the nucleic acid strand with the nucleic acid itself as a template.

As a preferred embodiment, the nucleic acid amplification is accelerated by introducing acceleration primers F2/R2 and/or LF/LR in the method for the synthesis of nucleic acids; wherein F2/R2 is a segment located on the 5' flank of the F1 region and the R1 region of the complementary strand of the original nucleic acid, and LF/LR is a middle segment located from the F1 region to the M1c region and from the M2 region to the R1.

The present invention also provides a nucleic acid, which consists of M1c, F1c, M, R1, and M2c regions in sequence in the 3' to 5' direction, wherein the M region comprises M1 and M2 regions, and the M2c region at the 5' end and the M1c region at the 3' end of the nucleic acid are complementary to M2 region and M1 region respectively; and a closed-loop structure is formed through annealing of the M2c region at the 5' end and the M1c region at the 3' end of the nucleic acid to the M region on the same strand.

The present invention provides a method for the synthesis of nucleic acids, which includes the steps of:
1-a) annealing a first oligonucleotide I to the F1c region of the template, wherein the template consists of an F1c region, an M region, and an R1 region in sequence in the 3' to 5' direction, wherein the M region comprises an M1 region and an M2 region, the first oligonucleotide I comprises an M1 region and an F1 region, the M1 region is linked to the 5' flank of the F1 region, wherein,
   F1 region: a region having a nucleotide sequence complementary to the Flc region,
   M1 region: a region having a nucleotide sequence identical to M1 in the M region;
1-b) synthesizing a first nucleic acid with the F1 region of the first oligonucleotide I as the starting point for synthesis; the first nucleic acid has a nucleotide sequence complementary to the template, the 5' end of the first nucleic acid has an M1 region capable of being annealed to an M1c region on the same strand, and a stem-loop can be formed by annealing the M1c region to the M1 region;
1-c) annealing a second oligonucleotide II to the Rlc region of the first nucleic acid under constant-temperature conditions, wherein the second oligonucleotide II comprises a R1 region and an M2c region, and the M2c region is linked to the 5' flank of the R1 region; wherein,
   R1 region: a region having a nucleotide sequence complementary to the Rlc region,
   M2c region: a region having a nucleotide sequence complementary to the M2 region in the M region; and
1-d) synthesizing a second nucleic acid with the R1 region of the second oligonucleotide II as the starting point for synthesis to obtain a target nucleic acid fragment.

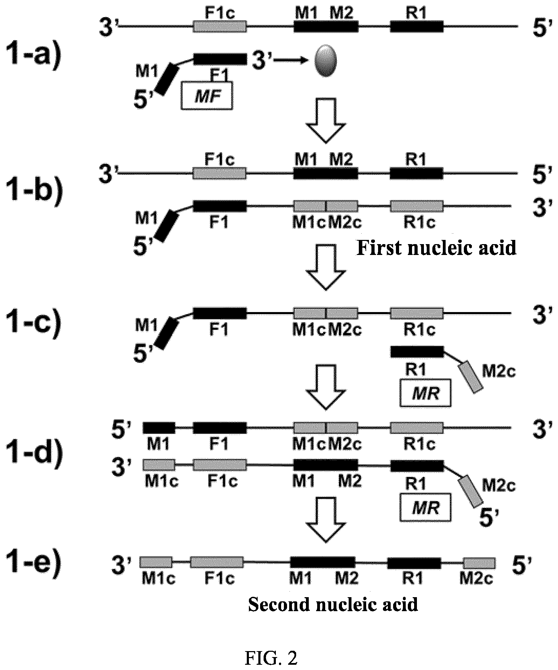

With reference to FIG. 2, which shows a schematic representation of the synthesis of nucleic acids (i.e., a second nucleic acid in the figure) of the present invention.

As a preferred embodiment, the template in step 1-a) is RNA, and the first nucleic acid in step 1-b) is synthesized by an enzyme having reverse transcriptase activity.

As a preferred embodiment, the nucleic acid fragments of the F1 region, the M region, and the R1 region are all 10-60 bp. Further preferably, the nucleic acid fragments of the F1 region, the M region, and the R1 region are all 20 bp.

The present invention also provides a kit for the synthesis of nucleic acids comprising the following components:
a first oligonucleotide I comprising an F1 region and an M1 region which is linked to the 5' flank of the F1 region, wherein
   F1 region: a region having a nucleotide sequence complementary to the Flc region,
   M1 region: a region having a nucleotide sequence identical to the M1 region in the M region;
a second oligonucleotide II comprising an R1 region and an M2c region which is linked to the 5' flank of the R1 region, wherein
   R1 region: a region having a nucleotide sequence complementary to the Rlc region,
   M2c region: a region having a nucleotide sequence complementary to the M2 region in the M region;
an enzyme for catalyzing the synthesis of nucleic acids; and
nucleotides which serve as substrates for the DNA polymerase.

As a preferred embodiment, the enzyme for catalyzing the synthesis of nucleic acids is a strand displacement DNA polymerase and/or a reverse transcriptase. Among them, the DNA polymerase comprises one or more of Bst DNA polymerase, Bca (exo-) DNA polymerase, DNA polymerase I Klenow fragment, Vent DNA polymerase, Vent (Exo-) DNA polymerase, Deep Vent DNA polymerase, Deep Vent (Exo-) DNA polymerase, Φ29 phage DNA polymerase, and MS-2 phage DNA polymerase, etc., Bst DNA polymerase or Bca (exo-) DNA polymerase is preferably used.

As a preferred embodiment, the kit further comprises an unwinding temperature regulator, preferably betaine.

As a preferred embodiment, the kit further comprises acceleration primers F2/R2 and/or LF/LR; wherein the F2/R2 is a segment located on the 5' flank of the F1 region and the R1 region of the complementary strand of the original nucleic acid, and the LF/LR is a middle segment located from the F1 region to the M1c region and from the M2 region to the R1.

As a preferred embodiment, the kit further comprises detection reagents for detecting products of nucleic acid synthesis reaction, preferably dyes having green excitation wavelengths and binding to all the minor groove regions of the dsDNA double helix, preferably SYBR Green I and EvaGreen.

As a preferred embodiment, the kit further comprises a buffer capable of maintaining an appropriate pH for the enzyme, a salt necessary for annealing or maintaining the catalytic activity of the enzyme, and a medium for protecting the enzyme.

The present invention also provides a use of the kit for non-diagnostic purposes in the synthesis of nucleic acids or in the detection of target nucleotide sequences in a sample. The present invention is suitable for the detection of various DNA and RNA, such as DNA and RNA from various animal and plant cells, bacteria, and viruses. For example, it is used for the detection of cDNA and RNA of H1 gene and N1 gene of H1N1 virus; for the detection of cDNA and RNA of MERS-CoV viruses, such as orfla and orflb segments of the RNA; for the detection of cDNA and RNA of SARS-CoV-2 viruses, such as the orf1a segment of the RNA; and for the detection of DNA of Cyprinid herpesvirus type II, etc.

Based on the method for the synthesis of nucleic acids under constant-temperature conditions of the present invention, there is provided a method for detecting a target nucleotide sequence in a sample, comprising performing amplification by the synthesis method of the present invention with a target nucleotide as a template and observing whether an amplified product is generated.

A probe comprising a nucleotide sequence complementary to the formed closed stem-loop structure is added to the amplified product, and hybridization between the two may be observed. The probe may also be labeled on a particle and an aggregation reaction by hybridization may be observed. The amplification method may be carried out in the presence of a nucleic acid detecting reagent, and the generation of amplified products is observed based on the change in signal.

Similar to the PCR amplification technique, based on the method for the synthesis of nucleic acids according to the present invention, there is further provide a method for detecting a mutation of a target nucleotide sequence in a sample, comprising performing amplification with the target nucleotide as a template by the method for the synthesis of nucleic acids under constant-temperature conditions according to the present invention. In the method, the synthesis of any complementary strand by the amplification method is impeded by the mutation to be amplified in the nucleotide sequence, and the generation of a signal is suppressed, thereby detecting the mutation.

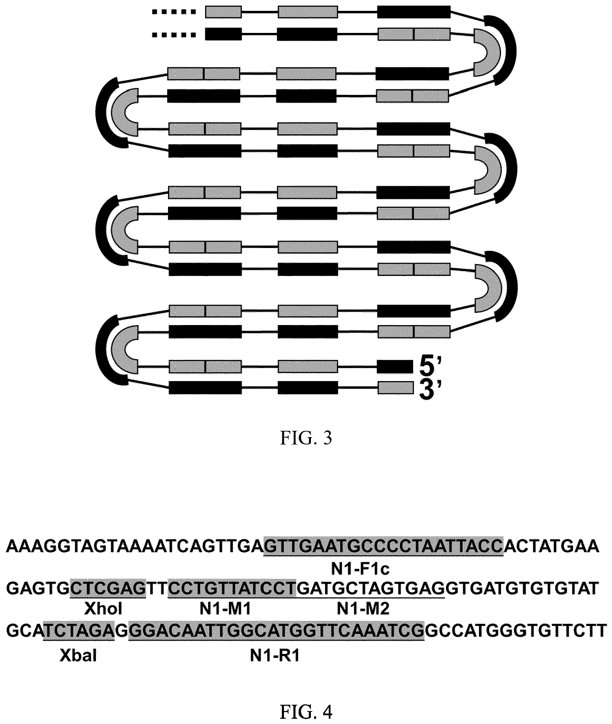

Nucleic acids synthesized by the present invention consist essentially of mutually complementary strands joined by a stem-forming loop structure. With reference to FIG. 3, which shows a schematic representation of an ideal amplified nucleic acid product formed by the synthetic methods of the present invention.

In general, a strand that cannot be separated into two or more molecules upon separation of complementary paired bases is referred to as a single strand. Base pairing may form in the complementary nucleotide sequences in the same strand; in the present invention, an intramolecular base-paired product can be obtained by allowing nucleic acids having nucleotide sequences joined end-to-end in a single strand to be base-paired within the same strand, and the product comprises a region constituting a distinct duplex and a loop not involved in the base pairing.

The nucleic acid having a nucleotide sequence of a closed stem-loop structure of the present invention can be defined as a single-stranded nucleic acid, comprising a complementary nucleotide sequence capable of annealing in the same strand. Nucleotides with complementary nucleotide sequences may be annealed to loops that do not involve base pairing. The loop-forming sequence may be any nucleotide sequence. The loop-forming sequence is capable of base pairing to initiate the synthesis of a complementary strand for displacement. The sequences different from those nucleotide sequences located in other regions are preferably provided to achieve specific annealing.

In the present invention, substantially identical nucleotide sequences are defined as follows: a sequence is substantially identical to a target nucleotide sequence when a complementary strand that is synthesized with this sequence as a template is annealed to the target nucleotide sequence to serve as the starting point for the synthesis of the complementary strand. For example, a sequence identical to F1 includes not only a sequence identical to F1 but also a nucleotide sequence that can serve as a template, which can give a nucleotide sequence that anneals to F1 and can serve as the starting point for the synthesis of a complementary strand. The term "annealing" according to the present invention refers to the formation of a nucleic acid of complementary structure based on Watson-Crick's base pairing. Thus, even if the nucleic acid strand that makes up base pairing is single strand, annealing also occurs if there is base pairing between complementary nucleotide sequences within the single-stranded molecule. The meanings expressed by annealing and hybridization in the present invention have overlapping parts, both of which involve base-pairing nucleic acids to form a double-stranded structure.

The number of nucleotide sequence pairs constituting the nucleic acid in the present invention is at least 1. In the desired model of the present invention, the number of nucleotide sequence pairs may be an integer multiple of one. In this case, there is no theoretical upper limit to the number of complementary nucleotide sequence pairs constituting the nucleotides in the present invention, and in the case of the synthesized nucleic acid product of the present invention consisting of a plurality of sets of complementary nucleotide sequences, the nucleic acid consists of repeated identical nucleotide sequences.

The single-stranded nucleic acid having a nucleotide sequence of the stem-loop structure synthesized according to the present invention has a structure different from that of a naturally occurring nucleic acid, and a nucleic acid derivative can also be synthesized by the method of the present invention using a nucleotide derivative as a substrate. The nucleotide derivatives include radioisotope-labeled nucleotides or binding ligands-, such as biotin- or digoxigenin-labeled nucleotide derivatives. These nucleotide derivatives can be used to label nucleic acids products. If the substrate is a fluorescent nucleotide, the nucleic acid product is a fluorescent derivative.

The synthesis of nucleic acids having the above structure can be initiated using DNA polymerase, which has strand displacement activity and the activity of initiating the annealing of the M1c region to the M1 region to synthesize a complementary strand.

The nucleic acids in the present invention generally include both DNA and RNA, and nucleic acids or modified nucleic acids from natural DNA or RNA in which the nucleotides are replaced by artificial derivatives are also included in the scope of the nucleic acids of the present invention. Typically, the nucleic acids of the present invention are comprised in biological samples, including tissues, cells, cultures, and secretions of animals, plants or microorganisms, and extracts thereof. The biological samples according to the present invention include genomes DNA or RNA from intracellular parasite, such as viruses or mycoplasma. The nucleic acids of the present invention are typically derived from the nucleic acids contained in the biological sample. Typical examples of nucleic acids synthesized according to the present invention include, for example, cDNA synthesized by mRNA, microRNA, etc., and nucleic acids amplified based on nucleic acids derived from biological samples.

The nucleic acids of the present invention are characterized in that the M1c and M2c regions at the 5' end and the 3' end of the nucleic acid are annealed to the M region on the same strand to form a closed loop, and the elongation can only be achieved under the action of DNA polymerase when M1c region at the 3'-end is annealed to the M region.

In the present invention, an oligonucleotide is a nucleotide that satisfies two requirements, i.e., they must be able to form complementary base pairing and supply an -OH group at the 3' end to serve as the starting point for complementary strand synthesis. Thus, the backbone of oligonucleotide need not be limited to phosphodiester linkage. For example, the oligonucleotide may have a backbone consisting of phosphorothioate derivatives in which O is replaced by S, or maybe peptide nucleic acids based on peptide linkages. Base refers to those bases that are capable of complementary pairing. Naturally occurring bases include five species of bases, i.e., A, C, T, G, and U, and the bases may also be analogs such as bromodeoxyuridine. Preferably, the oligonucleotide of the present invention can be used not only as the starting point of synthesis but also as a template for the synthesis of a complementary strand. The term polynucleotide of the present invention includes oligonucleotides. As used herein, the term "polynucleotide" is not limited in chain length, and the term "oligonucleotide" as used herein refers to a polymer of nucleotides having a relatively short chain length.

The oligonucleotide strands of the present invention have a length capable of base pairing with a complementary strand and maintaining a certain specificity under given conditions in the synthesis of various nucleic acids described below. Specifically, it consists of 5-200 bases, more preferably 10-50 base pairs. The known polymerase is recognized to have a chain length of at least 5 bases. The polymerase catalyzes sequence-dependent nucleic acid synthesis reaction. Therefore, the chain length of the annealed part should be longer than such length. In addition, a length of 10 bases or more is statistically desired to achieve target nucleotide specificity. On the other hand, the above chain lengths are examples of desirable ranges because of the difficulty of preparing a too-long nucleotide sequence by chemical synthesis. Exemplary chain lengths refer to that of a moiety annealing to a complementary strand. As described below, the oligonucleotides of the present invention can eventually anneal to at least two regions, respectively. Thus, the exemplary chain lengths herein are to be understood as the chain length of each region making up the oligonucleotide.

In addition, the oligonucleotides of the present invention may be labeled with known labels. Labels include binding ligands such as digoxin and biotin, enzymes, fluorophores, illuminants, and radioisotopes. The technique of replacing bases constituting oligonucleotides by fluorescent analogs is well known (WO95/05391, Proc. Natl. Acad. Sci. USA, 91, 6644-6648, 1994).

Other oligonucleotides of the present invention may also be bound to a solid phase. Alternatively, any portion of the oligonucleotide can be labeled with a binding ligand, such as biotin, and indirectly immobilized by a binding ligand, such as immobilized avidin. Where the immobilized oligonucleotide is the starting point of synthesis, the product nucleic acid of the synthetic reaction is captured by a solid phase, which facilitates its separation. The isolated moieties can be detected by nucleic acid-specific indicators or by hybridization to labeled probes. Target nucleic acid fragments of the nucleic acid products obtained by the present method can be recovered by digestion of the products with a restriction enzyme.

As used herein, the term "template" refers to a nucleic acid that serves as a template for the synthesis of a complementary strand. A complementary strand having a nucleotide sequence complementary to the template means a strand corresponding to the template. However, their relationship is only relative. That is, the synthesized complementary strand may serve as a template again. That is, the complementary strand may also serve as a template.

In the present invention, if RNA is targeted, the reaction can be performed only by additionally adding reverse transcriptase. That is, using RNA as a template, a complementary strand is synthesized by annealing of F1 to Flc in the template with the reverse transcriptase. When the synthesis of the complementary strand using DNA as a template is carried out with the reverse transcriptase, all the reactions for the synthesis of the complementary strand by the reverse transcriptase include the synthesis of the complementary strand using R1 annealed to Rlc as the starting point of the synthesis, and the complementary strand serves as a template in the strand displacement reaction. The preferred mode of the present invention as described above is a mode in which the first single-stranded nucleic acid is obtained by with RNA as a template. On the other hand, if a DNA polymerase such as Bca DNA polymerase and WarmStart^{®} RTx reverse transcriptase, etc. having both strand displacement activity and reverse transcriptase activity is used, not only synthesis from the first single-stranded nucleic acid of RNA but also subsequent reaction with DNA as a template can be performed similarly by the same enzyme.

The reaction is carried out in the presence of the following components: a buffer capable of maintaining an appropriate pH for the enzyme, a salt necessary for annealing or maintaining the catalytic activity of the enzyme, a medium for protecting the enzyme, and a regulator necessary for regulating the unwinding temperature (Tm). Tris-HCl having buffering action in the neutral or weak alkaline range is used as a buffer. The DNA polymerase is used to adjust the pH; appropriate amounts of KCl, NaCl, (NH₄)₂SO₄, etc. are used as salts to maintain the activity of the enzyme and regulate the unwinding temperature (Tm) of the nucleic acid; and bovine serum albumin or saccharides are used as the medium for protecting the enzyme. In addition, dimethyl sulfoxide (DMSO) or formamide are commonly used as regulators for unwinding temperature (Tm). The annealing of the oligonucleotide is regulated under defined temperature conditions using a regulator for unwinding temperature (Tm). Furthermore, betaine (N, N, N-trimethylglycine) or tetraalkylammonium salts is also effective in improving the efficiency of chain displacement through their isostabilization. The amplification of nucleic acid can be promoted by adding 0.2-3.0 M betaine, preferably 0.5-1.5 M betaine, to the reaction solution. Because these regulators for unwinding temperature have the effect of lowering the unwinding temperature, and appropriate stringency and reactivity conditions are empirically determined in conjunction with salt concentration, reaction temperature, and the like.

An important feature of the present invention is that a series of reactions cannot proceed unless the positional relationship of many regions is maintained. Due to this feature, non-specific synthesis reactions accompanied by non-specific synthesis of complementary strands are effectively prevented. Thus, the method of the present invention has a higher specificity when used for detection purposes.

Nucleic acids synthesized by the present invention are single strands, most of which may constitute complementary nucleotide sequences by base pairing. With this feature, the synthesized product can be detected. In the implementation of the method for synthesizing nucleic acid of the present invention, a fluorchrome can be used as a double-specific intercalator, such as ethidium bromide, SYBR Green I, Pico Green, or EvaGreen, and an increase in the intensity of fluorescence can be observed as the product increases during the synthesis of nucleic acid. By monitoring the fluorescence intensity, the progress of the real-time synthesis reaction can be tracked in a closed system. It is also contemplated to use this type of detection system in the PCR method, but there are a number of problems including the inability to distinguish the product signal and the primer dimer signal, etc. However, when this system is applied in the present invention, the ability to increase non-specific base pairing is very low, so it is expected that both high sensitivity and low interference would be achieved, and the detection can be achieved by the transfer of fluorescence energy in the same system, similarly to the application of double-specific intercalator.

In the method for the synthesis of nucleic acids in the present invention, a complementary chain reaction is catalyzed using an enzyme for catalyzing the synthesis of nucleic acids, which enzyme may be a DNA polymerase or reverse transcriptase, etc. Among them, the DNA polymerase can be selected from the following types of enzymes. In addition, various mutants of these enzymes, which have both sequence-dependent activity and strand displacement activity for complementary strand synthesis, can also be used in the present invention.
Bst DNA polymerase
Bst DNA polymerase (large fragment)
Bst 2.0 DNA polymerase
Bst Warmstart 2.0 DNA polymerase
Bst 3.0 DNA polymerase
Bca (exo-) DNA polymerase
DNA polymerase I Klenow fragment
Vent DNA polymerase
Vent (Exo-) DNA polymerase (Vent DNA polymerase lacking exonuclease activity)
Deep Vent DNA polymerase
Deep Vent (Exo-) DNA polymerase (Deep Vent DNA polymerase lacking exonuclease activity)
Φ29 phage DNA polymerase
MS-2 phage DNA polymerase
OmniAmp DNA polymerase

Among these enzymes, Bst DNA polymerase, Bca (exo-) DNA polymerase, and OmniAmp DNA polymerase are preferably used because of their good thermostability and high catalytic activity. In a preferred embodiment, the reaction of the present invention can be carried out under constant-temperature conditions, but it is not always possible to maintain the stability of the enzyme using constant-temperature conditions due to adjustment of unwinding temperature (Tm) and the like. Thus, the enzymes used require good thermostability.

Various reagents necessary for the synthesis or amplification of nucleic acids in the present invention may be prepackaged and provided in kit form. Specifically, a kit provided by the present invention comprises various oligonucleotides necessary as a primer for the synthesis of a complementary strand and an outer primer for a displacement reaction, a substrate dNTP for the synthesis of a complementary strand, a DNA polymerase for effecting strand displacement-type complementary strand synthesis, a buffer for providing appropriate conditions for an enzymatic reaction, and a medium necessary for detecting a product of the synthesis reaction. Specifically, in a preferred mode of the present invention, all of the reaction reagents provided have been added in advance, so that the reaction can be initiated by simply adding the sample. By using a system in which a reaction product can be detected in a container using a visible light signal or a fluorescent signal, it is not necessary to open and close the container after the reaction, which is very advantageous for preventing contamination.

The present invention synthesizes a single-stranded nucleic acid having a nucleotide sequence with a closed stem-loop structure, thereby initiating polynucleotide synthesis of the stem-loop structure. The nucleic acid has the following uses:
According to a preferred mode of the present invention, a large number of loops capable of base pairing are generated in a single-stranded nucleic acid. This means that a large number of probes can be hybridized to one molecule of nucleic acid to achieve high-sensitivity detection. Therefore, not only the sensitivity can be improved, but also the detection of nucleic acid can be realized based on a special reaction principle such as aggregation. For example, probes immobilized on fine particles (such as polystyrene latex or gold silver platinum nanoparticles, or two-dimensional nanomaterials) are added to the reaction product of the present invention to observe the aggregation of the latex or the like. The intensity of aggregation may be observed with high sensitivity and quantificationally by optical measurements. The aggregation can also be observed by the naked eye to establish a reaction system without an optical measuring device.

In addition, the reaction products of the present invention allow for some bindable labels, wherein each nucleic acid molecule can be subjected to chromatographic detection. In the field of immunoassays, analytical methods using chromatographic media with visible detection labels (immunochromatography) are mainly based on the principle that an analyte is sandwiched between an antibody immobilized on a chromatographic medium and a labeled antibody. The reaction products of the present invention allow this principle to be applied to the nucleic acid analysis. That is, a labeled probe for the cyclic moiety is prepared and immobilized on the chromatographic medium, allowing to perform analysis in the chromatographic medium. Capture probes complementary to the loop moiety are utilized, and because the reaction products of the present invention have a large number of single-stranded loops and the products bind to a large number of labeled probes, the detection of a visually recognizable signal is achieved.

On the other hand, a large number of loops of the reaction product of the present invention can be used as probes; for example, the probes are densely packed in a limited area in a DNA chip, whereas the number of oligonucleotides that can be immobilized in a certain area is limited in this technique, so that a large number of probes from the product of the present invention that can be annealed can be immobilized in a high density, i.e., the reaction products of the present invention can be used as immobilized probes on a DNA chip, and the amplified reaction products can be immobilized by any technique known in the art, or the immobilized oligonucleotides are used as oligonucleotides for the amplification reaction in the present invention, resulting in the formation of immobilized reaction products. Thus, by using the immobilized probes, hybridization of a large number of samples DNA is accomplished in a limited area, resulting in high signal values.

### BRIEF DESCRIPTION OF DRAWINGS

FIG. 1 is a schematic representation of the steps of a method for the synthesis of nucleic acids under constant-temperature temperature in the present invention.
FIG. 2 is a schematic representation of the steps of a method for the synthesis of nucleic acids provided by the present invention; wherein MF refers to the first oligonucleotide I and MR refers to the second oligonucleotide II.
FIG. 3 is a schematic diagram of an ideal amplified product formed by the synthetic method of the present invention.
FIG. 4 shows the positional relationship of each corresponding nucleotide sequence region in the H1N1 target nucleotide sequence of Example 1 in the present invention.
FIG. 5 is a photograph showing the result of agarose electrophoresis of the product obtained by the synthesis method of the single-stranded nucleic acid of the present invention with H1N1 as a template in Example 1 of the present invention; wherein Lane 1: Takara DL2,000 DNA Marker; Lane 2: 1 fmol H1N1 dsDNA.
FIG. 6 is a photograph showing the result of agarose gel electrophoresis of the product digested by the restriction enzyme in Example 1, wherein the product is obtained in Example 2 through the nucleic acid synthesis reaction of the present invention. wherein,
   Lane 1: molecular weight marker DNA ladder
   Lane 2: purified products digested with XbaI
   Lane 3: purified products digested with XhoI
   Lane 4: purified products digested with XhoI plus XbaI
   Lane 5: purified products
FIG. 7 is a real-time fluorescence curve during H1N1 target nucleotide sequence DNA amplification under the action of the primers in Example 3 of the present invention.
FIG. 8 is a graph showing the color change-based end-point monitoring of the reaction with HNB during H1N1 target nucleotide sequence DNA amplification under the action of the primers in Example 4 in the present invention.
FIG. 9 is a real-time fluorescence curve during H1N1 target nucleotide sequence in vitro transcribed RNA amplification under the action of the primers in Example 5 of the present invention.
FIG. 10 is a real-time fluorescence curve during MERS-orfl a target nucleotide sequence DNA amplification under the action of the primers in Example 6 of the present invention.
FIG. 11 is a schematic diagram showing the action site of the amplification principle of a target nucleotide with the addition of acceleration primers in Example 7 of the present invention.
FIG. 12 shows a plot of fluorescence intensity versus reaction time in the amplification reaction of MERS-orfl a system under the action of different combinations of acceleration primer in Example 7 of the present invention.
FIG. 13 shows a real-time fluorescence curve during the amplification of target nucleotide sequence DNA containing Cyprinid herpesvirus type II under the action of the Cyprinid herpesvirus type II target nucleotide primers in Example 8 of the present invention.
FIG. 14 shows a real-time fluorescence curve during the amplification of target nucleotide sequence DNA containing SARS-CoV-2-orfla gene under the action of the orf1a target nucleotide primers for novel coronavirusSARS-CoV-2 designed in Example 9 of the present invention.

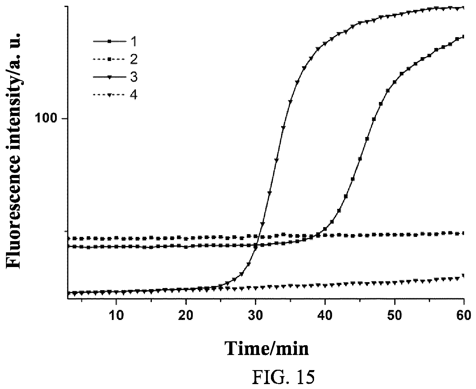

FIG. 15 shows real-time fluorescence curves during the amplification of target nucleotide sequence DNA containing the same concentration of MERS-orfl a gene under the action of different primers of MERS-orfl a target nucleotide sequence DNA in the present invention and CAMP technique designed in Example 10 of the present invention; wherein curve 1 is a real-time amplification plot of MERS-orfla target nucleotide by CAMP technique; curve 2 is a real-time amplification plot of a control without the MERS-orfl a target nucleotide by CAMP technique; curve 3 is a real-time amplification plot of MERS-orfla target nucleotide by the nucleic acid synthesis method of the present invention; and curve 4 is a real-time amplification plot of a control without the MERS-orfla target nucleotide by the nucleic acid synthesis method of the present invention.

### DETAILED DESCRIPTION

The experimental methods used in the following examples are all conventional unless otherwise specified, and can be specifically performed according to the specific methods listed in the manual: PCR Technology Experiment Guide (2nd edition), or according to the kit and product instructions; and materials, reagents and the like used in the following examples are commercially available unless otherwise specified.

### Example 1 Amplification of fragments in influenza A virus H1N1

Influenza A (H1N1) virusbelongs to the family Orthomyxoviridae and genus Influenzavirus A and causes symptoms such as fever, cough, fatigue, and inappetence in people, which are similar to those caused by common cold. In 2009, H1N1 influenza virus emerged to cause a pandemic, leading to a state of panic. The nucleic acid of H1N1 was generally detected by reverse transcription followed by detection of the cDNA using PCR techniques. Therefore, the new primers designed by the method of the present invention can also be applied in the detection of H1N1 virus. The nucleic acid of the invention was prepared by using artificially designed H1N1 inserted with enzyme cutting sites (from GenBank: GQ 290690.1) as a template. The two primers used in the experiments were N1-MF (nucleotide sequence as shown in SEQ ID NO.1) and N1-MR (nucleotide sequence as shown in SEQ ID NO. 2). These were designed to anneal to loop regions by use of adjacent stacking phenomenon. Furthermore, these primers are set to a high concentration such that annealing of N1-MF (or N1-MR) occurs preferentially.

M1c and M2c segments were synthesized at both ends of the target nucleotide H1N1 with the primers N1-MF and N1-MR, competed with the M segment on the target nucleotide to form a closed stem-loop structure. A combination of reaction solutions for the method for the synthesis of nucleic acids of the present invention with these primers was shown below.

The combination of reaction solutions was as follows, supplementing to 25 µL with ddH₂O:
20 mM Tris-HCl pH8.8
10 mM KCl
10 mM (NH₄)₂SO₄
14 mM MgSO₄
0.1 % Triton X-100
1 M Betaine
1.25 mM dNTP
8 U *Bst* DNA polymerase (NEW ENGLAND Biolabs)

### Primers:

1600 nM N1-MF
1600 nM N1-MR

Target nucleic acids: H1N1 dsDNA (nucleotide sequence as shown in SEQ ID NO. 3). FIG. 4 shows a positional relationship of each corresponding nucleotide sequence region in the H1N1 target nucleotide sequence.

The mixture was allowed to react at 63°C for 1 hour, after which the reaction was terminated at 80°C for 10 minutes, and then re-transferred to water pre-cooled with ice.

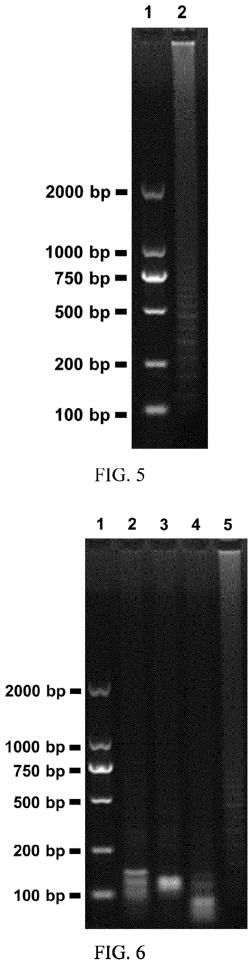

Confirmation of reaction: 1 µL of conventional nucleic acid electrophoresis loading buffer (kindly gifted by Takara DNA ladder) was added to 5 µL of the reaction solution after the reaction was terminated above, and the sample was electrophoresed on a GelRed (Biotum) prestained 1% agarose gel (dissolved in TAE) at 90 mV for 1 hour. Takara DL2,000 DNA Marker was used as a molecular weight marker. The gel after electrophoresis was used to verify the nucleic acid synthesized by the reaction. The results are as shown in FIG. 5, which is a photo of agarose electrophoresis of the product obtained with H1N1 as a template; wherein, Lane 1: Takara DL2,000 DNA Marker; Lane 2: 1 fmol H1N1 dsDNA. The results showed that a nucleic acid product with a broad molecular weight distribution was obtained, which verified that a super-large nucleic acid molecule may be obtained by the infinite self-assembly, annealing, and extension of the nucleic acid obtained by the method of the present invention.

### Example 2 Confirmation of the reaction products in Example 1 by restriction enzyme digestion

To verify that the nucleic acid obtained in Example 1 of the present invention had a structure in which the complementary nucleotide sequence was linked within a single strand in a loop structure, the product was digested with a restriction enzyme. The synthetic product of Example 1 may be presumed to be a nucleic acid with complementary sequences alternately linked within a single strand, if a theoretical fragment can be generated by digestion, and at the same time, there was no unclear flaky band pattern and non-electrophoretized band at a high molecular weight.

The reaction solution after the termination of the reaction in Example 1 was precipitated and purified by the precipitation action with ethanol, the resulting precipitate was recovered and redissolved in ultrapure water, the solution was digested at 37°C for 2 hours first with restriction enzymes XbaI, XhoI, respectively, and then with the combination of the two restriction enzymes, and the sample was electrophoresed on a GelRed (Biotum) prestained 1% agarose gel (dissolved in TAE) at 90 mV for 1 hour. Takara DL2,000 DNA Marker was used as a molecular weight marker. The nucleic acid was verified by the electrophoresed gel. As shown in FIG. 6, the results indicated that: the large nucleic acid fragment obtained was enzyme-digested into a small fragment, demonstrating that the product was obtained by amplifying the target nucleic acid, and no non-specific amplification occurs, demonstrating the specificity of the method of the present invention and that the nucleic acid products were alternately linked with complementary sequences.

### Example 3 Validation of H1N1 gene amplification reaction products using EvaGreen

EvaGreen, like SYBR Green I, is a dye having a green excitation wavelength and binding to all dsDNA double helix minor groove regions, and its inhibition of nucleic acid amplification reactions such as PCR is much less than the latter. In the free state, EvaGreen emits weak fluorescence, but upon binding to double-stranded DNA, the fluorescence is greatly enhanced. Therefore, the fluorescent signal intensity of EvaGreen is related to the amount of double-stranded DNA, and the amount of double-stranded DNA present in the nucleic acid amplification system can be detected according to the fluorescent signal.

A combination of reaction solutions for the method for the synthesis of nucleic acids of the present invention with the primers N1-MF (nucleotide sequence as shown in SEQ ID NO.1) and N1-MR (nucleotide sequence as shown in SEQ ID NO.2) was as follows.

The combination of reaction solutions was as follows, supplementing to 25 µL with ddH₂O:
20 mM Tris-HCl pH8.8
10 mM KCl
10 mM (NH₄)₂SO₄
14 mM MgSO₄
0.1 % Triton X-100
1 M Betaine
1.25 mM dNTP
8 U Bst DNA polymerase (NEW ENGLAND Biolabs)
1 X EvaGreen (Biotum)

### Primers:

1600 nM N1-MF
1600 nM N1-MR

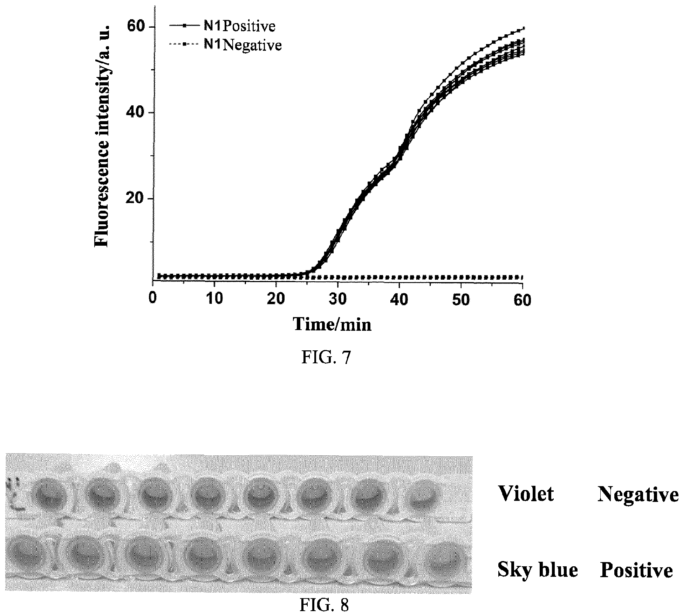

Target nucleic acids: H1N1 dsDNA (nucleotide sequence as shown in SEQ ID NO. 3). 8 positive controls and 8 negative controls were set for the amplification reaction. The constant Roche LC480 real-time PCR reaction temperature was constantly set at 63 °C and the reaction time was 60 min. FIG. 7 is a plot of fluorescence intensity versus reaction time. The application of fluorescence detection therein can realize real-time monitoring. As known from the real-time amplification curve: the fluorescence intensity gradually increased after 25 minutes of reaction, indicating that the synthesized nucleic acid products continued to elongate and form alternately linked complementary sequences.

### Example 4 Amplification reaction endpoint monitoring using hydroxynaphthol blue (HNB)

Hydroxynaphthol blue (HNB) belongs to the metal ion indicator, which is aimed to present different indication colors with the change of the amount of magnesium ion or manganese ion combined with by-product pyrophosphate in the reaction, so as to judge the result.

A combination of reaction solutions for the method for the synthesis of nucleic acids of the present invention with the primers N1-MF (nucleotide sequence as shown in SEQ ID NO.1) and N1-MR (nucleotide sequence as shown in SEQ ID NO.2) was as follows.

The combination of reaction solutions was as follows, supplementing to 25 µL with ddH₂O:
20 mM Tris-HCl pH8.8
10 mM KCl
10 mM (NH₄)₂SO₄
14 mM MgSO₄
0.1 % Triton X-100
1 M Betaine
1.25 mM dNTP
8 U Bst DNA polymerase (NEW ENGLAND Biolabs)
120 µM HNB

### Primers:

1600 nM N1-MF
1600 nM N1-MR

Target nucleic acids: H1N1 dsDNA (nucleotide sequence as shown in SEQ ID NO. 3). 8 positive controls and 8 negative controls were set for the amplification reaction. The constant reaction temperature in the thermostatic water bath was set at 63 °C and the reaction time was 60 min. FIG. 8 shows the results of the negative and positive reaction endpoint, wherein a color of violet indicates negative and sky blue indicates positive. The experimental results show that the application of HNB therein can judge the reaction results by color, and the judgment can be carried out without instrument assistance.

### Example 5 Amplification of RNA target gene using EvaGreen-based real-time fluorescence

cDNA can be synthesized by AMV reverse transcriptase with RNA as a template, and the RNA can be detected by combining with Bst DNA polymerase.
cDNA was synthesized with primers N1-MF (nucleotide sequence as shown in SEQ ID NO. 1) and N1-MR (nucleotide sequence as shown in SEQ ID NO.2) with RNA as a template. The combination of the reaction solution was as follows, supplementing to 25 µL with ddH₂O.
20 mM Tris-HCl pH8.8
10 mM KCl
10 mM (NH₄)₂SO₄
14 mM MgSO₄
0.1 % Triton X-100
1 M Betaine
1.25 mM dNTP
8 U *Bst* DNA polymerase (NEW ENGLAND Biolabs)
5 U AMV reverse transcriptase
1 X EvaGreen (Biotum)

### Primers:

1600 nM N1-MF
1600 nM N1-MR

Target nucleic acids: H1N1 RNA (nucleotide sequence as shown in SEQ ID NO. 4). The H1N1 RNA was obtained by transcribing H1N1 dsDNA (nucleotide sequence as shown in SEQ ID NO.3) in vitro.

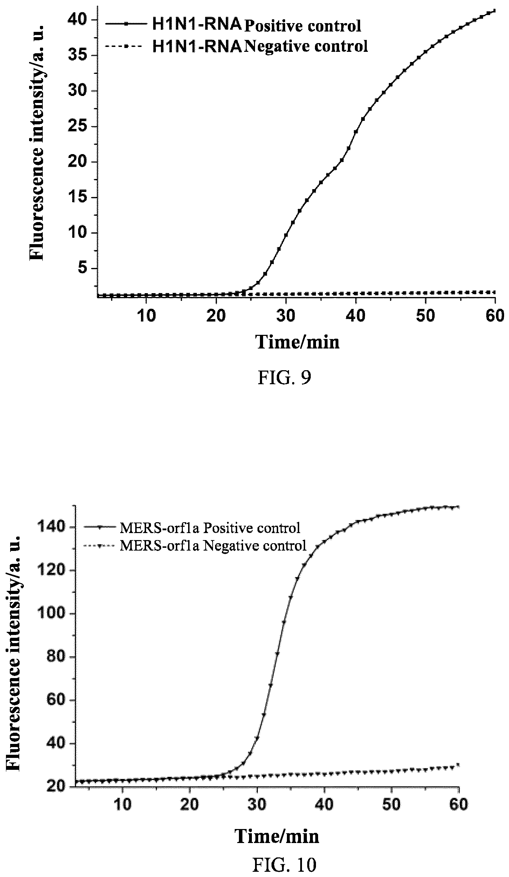

The constant Roche LC480 real-time PCR reaction temperature was set at 63 °C and the reaction time was 60 min. FIG. 9 is a plot of fluorescence intensity versus reaction time. This result shows that the present method is also feasible for RNA detection.

### Example 6 Amplification of MERS-orfl a fragments using EvaGreen-based real-time fluorescence

The artificially designed MERS-orfla inserted with enzyme cutting sites (from GenBank: KX108946.1) was used as a template, and two used primers were Mo1aMF (nucleotide sequence as shown in SEQ ID NO.5) and Mo1aMR (nucleotide sequence as shown in SEQ ID NO. 6). These were designed to anneal to loop regions by adjacent stacking phenomenon. Furthermore, these primers were set to a high concentration such that annealing of Mo1aMF (or Mo1aMR) occurs preferentially.

A combination of reaction solutions for the method for the synthesis of nucleic acids of the present invention with these primers was shown below.

The combination of reaction solutions was as follows, supplementing to 25 µL with ddH₂O:
20 mM Tris-HCl pH8.8
10 mM KCl
10 mM (NH₄)₂SO₄
14 mM MgSO₄
0.1 % Triton X-100
1 M Betaine
1.25 mM dNTP
8 U *Bst* DNA polymerase (NEW ENGLAND Biolabs)
1 X EvaGreen (Biotum)

### Primers:

1600 nM Mo1aMF
1600 nM Mo1aMR

Target nucleic acids: MERS-orfl a dsDNA (nucleotide sequence as shown in SEQ ID NO. 7). The constant Roche LC480 real-time PCR reaction temperature was set at 63 °C and the reaction time was 60 min. A plot of fluorescence intensity versus reaction time is as shown in FIG. 10. This result indicates that the application of fluorescence detection therein can achieve real-time detection of the amplification of MERS-orfl a fragments.

### Example 7 Amplification of MERS-orfl a dsDNA target gene using acceleration primers

A combination of reaction solutions for the method for the synthesis of nucleic acids of the present invention with these primers was shown below.

The acceleration primer sets were divided into four groups, and the only difference is in terms of primer groups (wherein acceleration primer 1 comprises primers F2 and R2, and acceleration primer 2 comprises primers LF and LR):
a. without acceleration primer 1, without acceleration primer 2
b. with acceleration primer 1, without acceleration primer 2
c. without acceleration primer 1, with acceleration primer 2
d. with acceleration primer 1, with acceleration primer 2

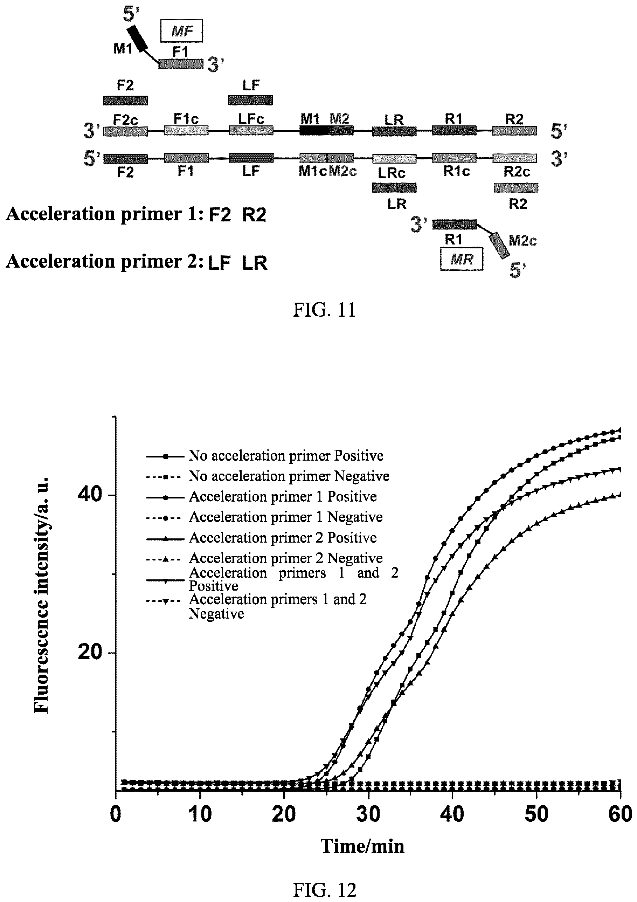

With reference to FIG. 11, which shows a schematic diagram showing the action site of the amplification principle of a target nucleotide with the acceleration primers in Example 7 of the present invention.

The combination of reaction solutions was as follows, supplementing to 25 µL with ddH₂O:
20 mM Tris-HCl pH8.8
10 mM KCl
10 mM (NH₄)₂SO₄
14 mM MgSO₄
0.1 % Triton X-100
1 M Betaine
1.25 mM dNTP
8 U *Bst* DNA polymerase (NEW ENGLAND Biolabs)
1 X EvaGreen (Biotum)

### Primer a:

1600 nM Mo1aMF (sequence as shown in SEQ ID NO.5)
1600 nM Mo1aMR (sequence as shown in SEQ ID NO.6)

### Primer b:

1600 nM Mo1aMF (sequence as shown in SEQ ID NO. 5)
1600 nM Mo1aMR (sequence as shown in SEQ ID NO. 6)
200 nM Mo1aF2 (sequence as shown in SEQ ID NO. 8)
200 nM Mo1aR2 (sequence as shown in SEQ ID NO. 9)

### Primer c:

1600 nM Mo1aMF (sequence as shown in SEQ ID NO. 5)
1600 nM Mo1aMR (sequence as shown in SEQ ID NO. 6)
800 nM Mo1aLF (sequence as shown in SEQ ID NO. 10)
800 nM Mo1aLR (sequence as shown in SEQ ID NO. 11)

### Primer d:

1600 nM Mo1aMF (sequence as shown in SEQ ID NO. 5)
1600 nM Mo1aMR (sequence as shown in SEQ ID NO. 6)
200 nM Mo1aF2 (sequence as shown in SEQ ID NO. 8)
200 nM Mo1aR2 (sequence as shown in SEQ ID NO.9)
800 nM Mo1aLF (sequence as shown in SEQ ID NO. 10)
800 nM Mo1aLR (sequence as shown in SEQ ID NO. 11)

Target nucleic acids corresponding to the primers of each of group a, b, c, and d were as follows: MERS-orfla dsDNA (sequence as shown in SEQ ID NO. 7). The constant Roche LC480 real-time PCR reaction temperature was set at 63 °C and the reaction time was 60 min. A plot of fluorescence intensity versus reaction time is as shown in FIG. 12. The resulting Ct values for groups a, b, c, and d were approximately 22 min, 23 min, 25 min, and 27 min, respectively. The Ct values of group a and group b were compared, indicating that acceleration primer 1 had an acceleration effect; the Ct values of group a and group c were compared, indicating that acceleration primer 2 had an acceleration effect; the Ct values of group a, group b, and group c were compared, indicating that acceleration primer 2 had better acceleration effect than acceleration primer 1; and the Ct values of group a, group b, group c, and group d were compared, indicating that acceleration primer 1 and acceleration primer 2 had a synergistic effect.

### Example 8 Amplification of target gene of Cyprinid Herpesvirus Type II

Koi herpesvirus disease and herpesviral hematopoietic necrosis disease caused by Cyprinid herpesvirus infection are serious threats to carp aquaculture. Cyprinid herpesvirus is a highly pandemic and contagious pathogen, causing a pandemic worldwide and showing 80%-100% mortality for the infected fish. The World Organization for Animal Health (OIE) highly concerned the disease and listed it as priority epidemics; meanwhile, the disease was also listed as Class II animal epidemics in China, and daily monitoring was carried out. It is very important to develop appropriate technique to achieve rapid detection of related epidemic diseases to respond to the epidemic situation in time. Thus CyHv-II was selected as a potential object for application of the methods of the present invention.

The combination of reaction solutions was as follows, supplementing to 25 µL with ddH₂O:
20 mM Tris-HCl pH8.8
10 mM KCl
10 mM (NH₄)₂SO₄
14 mM MgSO₄
0.1 % Triton X-100
1 M Betaine
1.25 mM dNTP
8 U *Bst* DNA polymerase (NEW ENGLAND Biolabs)
1 X EvaGreen (Biotum)

### Primers:

1600 nM CyHVII-MF (sequence as shown in SEQ ID NO. 12)
1600 nM CyHVII-MR (sequence as shown in SEQ ID NO. 13)
Target nucleic acids: CyHVIII dsDNA (sequence as shown in SEQ ID NO.14)

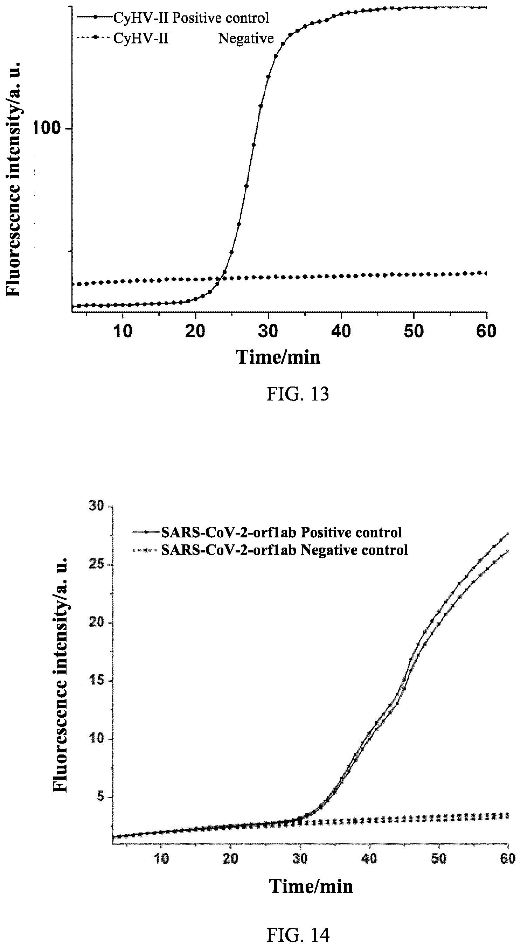

The constant Roche LC480 real-time PCR reaction temperature was set at 63 °C and the reaction time was 60 min. A plot of fluorescence intensity versus reaction time is as shown in FIG. 13. The amplification plot indicates that the method can be applied to the field for the detection and control of Cyprinid herpesvirus in aquaculture.

### Example 9 Amplification of fragments in SARS-CoV-2- ORF1ab

The artificially designed SARS-CoV-2-orfla inserted with enzyme cutting sites (from GenBank: NC_045512) was used as a template, and two used primers were SARS-CoV-2- O-MF (nucleotide sequence as shown in SEQ ID NO. 15) and SARS-CoV-2-O-MR (nucleotide sequence as shown in SEQ ID NO. 16). These were designed to anneal to loop regions by adjacent stacking phenomenon. Furthermore, these primers were set to a high concentration such that annealing of SARS-CoV-2- O-MF (or SARS-CoV-2- O-MR) occurred preferentially.

A combination of reaction solutions for the method for the synthesis of nucleic acids of the present invention with these primers was shown below.

The combination of reaction solutions was as follows, supplementing to 25 µL with ddH₂O:
20 mM Tris-HCl pH8.8
10 mM KCl
10 mM (NH₄)₂SO₄
14 mM MgSO₄
0.1 % Triton X-100
1 M Betaine
1.25 mM dNTP
8 U *Bst* DNA polymerase (NEW ENGLAND Biolabs)

### Primers:

1600 nM SARS-CoV-2- O-MF
1600 nM SARS-CoV-2- O-MR
Target nucleic acids: synthetic dsDNA of SARS-CoV-2-orf1a sequence (nucleotide sequence as shown in SEQ ID NO. 17).

Two groups of negative and positive controls were set. The constant Roche LC480 real-time PCR reaction temperature was set at 63 °C and the reaction time was 60 min. A plot of fluorescence intensity versus reaction time is as shown in FIG. 14. The amplification plot indicates that the method can be applied to the field for the detection of SARS-CoV-2.

### Example 10 Comparison with CAMP technique

The CAMP technique (Patent Application No. 201710828028.8) is an inventive approach for the synthesis of nucleic acid previously filed by the inventors. The present invention has the advantage of reducing the core primer length and increasing the reaction rate compared to the CAMP technique.

With the same target fragment MERS-orfla (from GenBank: KX108946.1) as an object template, the two primers used in the present invention are Mo1aMF (nucleotide sequence as shown in SEQ ID NO.5) and Mo1aMR (nucleotide sequence as shown in SEQ ID NO. 6), and the two primers used in the CAMP technique are Mo1aNF (nucleotide sequence as shown in SEQ ID NO. 18) and Mo1aMR (nucleotide sequence as shown in SEQ ID NO. 19).

A combination of reaction solutions for the method for the synthesis of nucleic acids of the present invention with these primers was shown below.

The combination of reaction solutions was as follows, supplementing to 25 µL with ddH₂O:
20 mM Tris-HCl pH8.8
10 mM KCl
10 mM (NH₄)₂SO₄
14 mM MgSO₄
0.1 % Triton X-100
1 M Betaine
1.25 mM dNTP
8 U *Bst* DNA polymerase (NEW ENGLAND Biolabs)
1 X EvaGreen (Biotum)

### Primers:

1600 nM Mo1aMF
1600 nM Mo1aMR

In the CAMP technique, a combination of reaction solutions for the method for the synthesis of nucleic acids of the present invention with CAMP primers was shown below.

The combination of reaction solutions was as follows, supplementing to 25 µL with ddH₂O:
20 mM Tris-HCl pH8.8
10 mM KCl
10 mM (NH₄)₂SO₄
14 mM MgSO₄
0.1 % Triton X-100
1 M Betaine
1.25 mM dNTP
8 U *Bst* DNA polymerase (NEW ENGLAND Biolabs)
1 X EvaGreen (Biotum)

### Primers:

1600 nM MolaNF
1600 nM MolaNR

Target nucleic acids: MERS-orfla dsDNA (nucleotide sequence as shown in SEQ ID NO. 7). The constant Roche LC480 real-time PCR reaction temperature was set at 63 °C and the reaction time was 60 min. A plot of fluorescence intensity versus reaction time is as shown in FIG. 15.

Compared with the primers Mo1aMF (nucleotide sequence as shown in SEQ ID NO.5) and Mo1aMR (SEQ ID NO.6) with lengths of 31 bp and 33 bp, respectively, the two primers used in the CAMP technique, MolaNF (nucleotide sequence as shown in SEQ ID NO. 18) and Mo1aMR (nucleotide sequence as shown in SEQ ID NO. 19) have lengths of 40 bp and 42 bp. It can be found that the length of primer used in the present invention is significantly shorter than that used in CAMP, which is very advantageous for saving primer cost in application.

Meanwhile, it can be observed from the real-time amplification plot, curve 1 is a real-time amplification plot of MERS-orfla target nucleotide by CAMP technique; curve 2 is a real-time amplification plot of a control without the MERS-orfla target nucleotide by CAMP technique; curve 3 is a real-time amplification plot of MERS-orfl a target nucleotide by the present invention; and curve 4 is a real-time amplification plot of a control without the MERS-orfla target nucleotide by the present invention. Curve 1 with a Ct value of about 38 min was compared with curve 3with a Ct value of about 25 min. It can be seen that the amplification rate of the present invention is significantly faster than that of the CAMP technique for the template of same concentration.

The above merely describes preferred examples of the present invention, and the present invention is not limited to the above examples. It will be apparent to those skilled in the art that various changes and modifications can be made within the spirit and scope of the present invention. Any changes and modifications are intended to be within the scope of the present invention.

## Claims

1. A method for the synthesis of nucleic acids under constant-temperature conditions for non-diagnostic purposes, **characterized by** comprising the steps of:
1) providing a nucleic acid consisting of M1c, F1c, M, R1, and M2c regions in sequence in the 3' to 5' direction, wherein the M region comprises M1 and M2 regions, and the M2c region at the 5' end and the M1c region at the 3' end of the nucleic acid are complementary to M2 region and M1 region respectively; and a closed-loop structure is formed through annealing of the M2c region at the 5' end and the M1c region at the 3' end of the nucleic acid to the M region on the same strand;
2) annealing a first oligonucleotide I to the Flc region of the nucleic acid provided in step 1), and then performing synthesis with the F1 region of the first oligonucleotide I as the starting point for synthesis; wherein the first oligonucleotide I comprises an M1 region and an F1 region;
3) annealing the M1c region at the 3' end of nucleic acid provided in step 1) to the M1 region, and synthesizing a complementary strand of the nucleic acid with the nucleic acid as a template, the synthesized nucleic acid sequence is referred to as nucleic acid A;
4) annealing a second oligonucleotide II to the Rlc region of the nucleic acid A provided in step 3), and then performing synthesis with the R1 region of the second oligonucleotide II as the starting point for synthesis; wherein the second oligonucleotide II comprises an R1 region and an M2c region; and
5) annealing the M region at the 3' end of nucleic acid A provided in step 3) to the adjacent Mc region, and synthesizing a complementary strand of the nucleic acid A with the nucleic acid A as a template;

2. The method for the synthesis of nucleic acids under constant-temperature conditions according to claim 1, **characterized in that** the resulting nucleic acid strand is capable of indefinite elongation by self-pairing, and the M1c region at the 3' end of the nucleic acid strand is paired with the complementary segment, the M1 region, on the strand to serve as the starting point for synthesis to continuously elongate the nucleic acid strand with the nucleic acid itself as a template.

3. The method for the synthesis of nucleic acids under constant-temperature conditions according to claim 1, **characterized in that** the nucleic acid amplification is accelerated by introducing acceleration primers F2/R2 and/or LF/LR in the method for the synthesis of nucleic acids; wherein F2/R2 is a segment located on the 5' flank of the F1 region and the R1 region of the complementary strand of the original nucleic acid, and LF/LR is a middle segment located from the F1 region to the M1c region and from the M2 region to the R1.

4. A nucleic acid, **characterized in that** the nucleic acid consists of M1c, F1c, M, R1, and M2c regions in sequence in the 3' to 5' direction, wherein the M region comprises M1 and M2 regions, and the M2c region at the 5' end and the M1c region at the 3' end of the nucleic acid are complementary to M2 and M1 regions respectively; and a closed-loop structure is formed through annealing of the M2c region at the 5' end and the M1c region at the 3' end of the nucleic acid to the M region on the same strand.

5. A method for the synthesis of the nucleic acid according to claim 4, comprising the steps of:
1-a) annealing a first oligonucleotide I to the F1c region of a template, wherein the template consists of a F1c region, an M region, and an R1 region in sequence in the 3' to 5' direction, wherein the M region comprises an M1 region and an M2 region, the first oligonucleotide I comprises an M1 region and an F1 region, the M1 region is linked to the 5' flank of the F1 region, wherein,
F1 region: a region having a nucleotide sequence complementary to the Flc region,
M1 region: a region having a nucleotide sequence identical to M1 in the M region;
1-b) synthesizing a first nucleic acid with the F1 region of the first oligonucleotide I as the starting point for synthesis; the first nucleic acid has a nucleotide sequence complementary to the template, the 5' end of the first nucleic acid has an M1 region capable of being annealed to an M1c region on the same strand, and a stem-loop can be formed by annealing the M1c region to the M1 region;
1-c) annealing a second oligonucleotide II to the Rlc region of the first nucleic acid under constant-temperature conditions, wherein the second oligonucleotide II comprises a R1 region and an M2c region, and the M2c region is linked to the 5' flank of the R1 region; wherein,
R1 region: a region having a nucleotide sequence complementary to the Rlc region,
M2c region: a region having a nucleotide sequence complementary to the M2 region in the M region; and
1-d) synthesizing a second nucleic acid with the R1 region of the second oligonucleotide II as the starting point for synthesis to obtain a target nucleic acid fragment.

6. The method for the synthesis of a nucleic acid according to claim 5, **characterized in that** the template in step 1-a) is RNA, and the first nucleic acid in step 1-b) is synthesized by an enzyme having reverse transcriptase activity.

7. The method for synthesis of a nucleic acid according to claim 5 or 6, **characterized in that** the nucleic acid fragments of the F1 region, the M region, and the R1 region are all 10-60 bp.

8. A kit for the synthesis of a nucleic acid comprising the following components:
a first oligonucleotide I comprising an F1 region and an M1 region which is linked to the 5' flank of the F1 region, wherein
F1 region: a region having a nucleotide sequence complementary to the Flc region,
M1 region: a region having a nucleotide sequence identical to the M1 region in the M region;
a second oligonucleotide II comprising an R1 region and an M2c region which is linked to the 5' flank of the R1 region, wherein
R1 region: a region having a nucleotide sequence complementary to the Rlc region,
M2c region: a region having a nucleotide sequence complementary to the M2 region in the M region;
an enzyme for catalyzing the synthesis of nucleic acids; and
nucleotides which serve as substrates for the DNA polymerase.

9. The kit according to claim 8, **characterized in that** the enzyme for catalyzing the synthesis of nucleic acids is a strand displacement DNA polymerase and/or a reverse transcriptase.

10. The kit according to claim 8, **characterized in that** the kit further comprises acceleration primers F2/R2 and/or LF/LR; wherein the F2/R2 is a segment located on the 5' flank of the F1 region and the R1 region of the complementary strand of the original nucleic acid, and the LF/LR is a middle segment located from the F1 region to the M1c region and from the M2 region to the R1.

11. The kit according to claim 8, **characterized in that** the kit further comprises detection reagents for detecting products of nucleic acid synthesis reaction, preferably dyes having green excitation wavelengths and binding to the minor groove region of the dsDNA double helix, preferably SYBR Green I and EvaGreen.

12. The kit according to claim 8, **characterized in that** the kit further comprises a buffer capable of maintaining an appropriate pH for the enzyme reaction, a salt necessary for annealing or maintaining the catalytic activity of the enzyme, and a medium protecting the enzyme.

13. Use for non-diagnostic purposes of the kit according to any of claims 8-12 in the synthesis of nucleic acids or in the detection of target nucleotide sequences in a sample.
